# EUROPEAN PATENT APPLICATION

(11) **EP 4 368 138 A1**
(43) Date of publication of application: **15.05.2024**
(21) Application number: 22315283.6
(22) Date of filing: 10.11.2022
(51) Int. Cl.: A61B 34/20

(54) **SYSTEM FOR DETECTING ELECTROMAGNETIC DISTURBANCES DURING A SURGERY**

(71) Applicant: MinMaxMedical, 38400 Saint-Martin-D'Hères (FR)
(72) Inventor: Larue, Jean-François, 38400 SAINT MARTIN D'HERES (FR)
(74) Representative: Regimbeau

(57) **Abstract**

The present invention relates to a system (100) for determining that an electromagnetic disturbance is impairing measurements made by an electromagnetic tracking unit (200) during a surgery, the system comprising
the electromagnetic tracking unit (200) configured to track relative poses of at least two bone portions linked to one another by at least one joint, based on measurements of an electromagnetic field emitted and received, respectively, by at least two electromagnetic transducers (201, 202) of the electromagnetic tracking unit (200),
at least one storage unit (301) in which at least one kinematic pattern of authorized relative displacements of the tracked bone portions with respect to one another is recorded, and
one data processor (302) in communication with the storage unit (301) and with the electromagnetic tracking unit (200),
wherein the data processor (302) is configured to
receive the tracked relative poses of the bone portions with respect to one another and compute a real-time kinematic of the bone portions,
compare the real-time kinematic of the bone portions with the recorded kinematic pattern,
determine a difference between the real-time kinematic and the recorded kinematic pattern,
determine that at least one of the measurements of the electromagnetic field is impaired when the difference exceeds a predetermined threshold.

## Description

The present invention relates to the field of electromagnetic tracking systems and particularly to electromagnetic tracking systems used during surgeries. Especially, the present invention relates to a system for detecting electromagnetic disturbances which can impair the tracking implemented through such electromagnetic tracking systems.

Electromagnetic tracking offers many advantages over optical tracking for surgical navigation and robotics. First, the electromagnetic tracking systems are not sensitive to line-of-sight issues as are the optical tracking systems. Second, the electromagnetic trackers - also called "electromagnetic transducers" in the present document - used in electromagnetic tracking systems are smaller than the optical trackers used in optical tracking systems. This size reduction permits to use a greater number of trackers, for instance one electromagnetic tracker can be attached to each bone involved in the surgery, thus improving the accuracy of the computed poses of the objects to which the trackers are attached.

However, one potential issue of the electromagnetic tracking systems is that electromagnetic measurements can be affected by the presence of disturbing materials in the vicinity of the system, such as metallic objects. The presence of metallic objects, and more generally of disturbing materials, is not obvious and it can lead to erroneous measurements but with no information that such measurements are impaired.

One solution to detect such errors is to place two small electromagnetic sensors on a unique bone that has to be tracked, at distant locations. As their relative pose should remain fix for the whole duration of the surgery, any variation in this relative pose can be interpreted as the sign that at least one of the measurements is erroneous. Unfortunately, implementing this solution adds an invasive and complex step difficult to perform.

Thus, there remains a need for improving the currently used electromagnetic tracking systems, and especially to improve the reliability of such systems.

The present invention aims at solving the described drawback by proposing a system for determining that an electromagnetic disturbance is impairing measurements made by an electromagnetic tracking unit during a surgery, the system comprising
- the electromagnetic tracking unit configured to track relative poses of at least two bone portions linked to one another by at least one joint, based on measurements of an electromagnetic field emitted and received, respectively, by at least two electromagnetic transducers of the electromagnetic tracking unit,
- at least one storage unit in which at least one kinematic pattern of authorized relative displacements of the tracked bone portions with respect to'one another is recorded, and
- one data processor in communication with the storage unit and with the electromagnetic tracking unit,
wherein the data processor is configured to
- receive the tracked relative poses of the bone portions with respect to one another and compute a real-time kinematic of the bone portions,
- compare the real-time kinematic of the bone portions with the recorded kinematic pattern,
- determine a difference between the real-time kinematic and the recorded kinematic pattern,
- determine that at least one of the measurements of the electromagnetic field is impaired when the difference exceeds a predetermined threshold.

The invention may be complemented by one or several of the following features, alone or in combination.

The system comprises at least one warning device in communication with the data processor, the data processor being further configured to trigger a warning signal generated by the warning device, when it is determined that at least one of the measurements of the electromagnetic field is impaired.

The electromagnetic tracking unit is configured to track the relative poses of at least three bone portions linked to one another by at least two joints, and wherein the data processor is further configured to determine which electromagnetic transducer gives impaired measurement(s).

The data processor is configured to determine which electromagnetic transducer gives impaired measurement(s) by
- artificially creating pairs of electromagnetic transducers, each pair of electromagnetic transducers comprising one shared electromagnetic transducer with at least one of the other pairs of electromagnetic transducers
- receiving the tracked relative poses of the bone portions with respect to one another and compute real-time kinematics of the bone portions,
- comparing the determined real-time kinematics with the recorded kinematic pattern of authorized displacements for each pair of electromagnetic transducers,
- determining a difference between the real-time kinematics and the recorded kinematic pattern for each pair of electromagnetic transducers,
- determining that the measurement of the electromagnetic field given by one pair of electromagnetic transducers is impaired when the difference exceeds a predetermined threshold,
- determining which electromagnetic transducer gives impaired measurements based on the determination of which pair of electromagnetic transducers gives impaired measurements.

The system comprises at least one display and wherein the data processor is configured to display which electromagnetic transducer gives impaired measurements.

The data processor is configured to compute a corrected relative pose of the bone portions by ignoring the impaired measurement(s). The data processor can also be configured to compute a corrected relative pose of the bone portions by replacing the received theoretical pose of the electromagnetic transducer which gives impaired measurements by its theoretical pose recorded with the kinematic pattern of authorized relative displacements.

The kinematic pattern of authorized relative displacements is recorded based on a 3D model of the tracked bone portions.

The kinematic pattern of authorized relative displacements is recorded as authorized relative displacements of the electromagnetic transducers.

The electromagnetic tracking unit comprises one electromagnetic transmitter adapted to emit the electromagnetic field and at least two electromagnetic receivers adapted to receive and measure the emitted electromagnetic field, each of the electromagnetic receivers being configured to be attached, respectively, to one of the tracked bone portions.

The invention further concerns a surgical robotic system comprising, at least
a robotic arm comprising at least one motorized joint, the robotic arm being configured to hold a surgical tool,
the surgical tool configured to treat a region of interest of an anatomical structure,
the system for determining that an electromagnetic disturbance is impairing measurements made by the electromagnetic tracking unit according to the invention.

According to an aspect of the invention, the data processor of the system for determining that an electromagnetic disturbance is impairing measurements made by the electromagnetic tracking unit can be further configured to trigger an emergency stop of the robotic arm when it is determined that at least one of the measurements of the electromagnetic field is impaired.

The present invention further relates to a method for determining that an electromagnetic disturbance is impairing measurements made by an electromagnetic tracking unit during a surgery, the method comprising the steps of
recording a kinematic pattern of authorized relative displacements of at least two bone portions linked to one another by at least one joint,
receiving relative poses of the at least two bone portions, these relative poses of the bone portions being tracked by the electromagnetic tracking unit,
computing a real-time kinematic of the bone portions,
comparing the real-time kinematic of the bone portions with the recorded kinematic pattern,
determining a difference between the real-time kinematic and the recorded kinematic pattern,
determining that at least one of the measurements of the electromagnetic field is impaired when the difference exceeds a predetermined threshold.

The invention may be complemented by one or several of the following features, alone or in combination.

The method further comprises a step of triggering a warning signal when the difference exceeds the predetermined threshold.

The tracked bone portions are two portions of a tibia, the joint being formed during an osteotomy surgery

The tracked bone portions are a femur and a tibia.

The tracked bone portions are adjacent vertebrae.

The step of recording the kinematic pattern of authorized relative displacements of the tracked bone portions comprises
acquiring a plurality of 2D images of the bone portions to track,
computing a 3D model of the bone portions to track based on the plurality of 2D images
simulating; virtually, authorized relative displacement of the bone portions to track and recording the simulation as the kinematic pattern of authorized relative displacements.

The step of recording the kinematic pattern of authorized relative displacements of the tracked bone portions comprises
attaching each electromagnetic transducer to one of the bone portions to track
applying a determined list of constraints to the tracked bone portions
recording a kinematic of the tracked bone portions while applying the constraints as the kinematic of authorized relative displacements.

The step of recording a kinematic pattern of authorized relative displacements is implemented for at least three bone portions linked to one another by at least two joints, the method further comprising the step of determining which electromagnetic transducer(s) gives impaired measurement.

The step of determining which electromagnetic transducer(s) gives impaired measurement comprises
artificially creating pairs of electromagnetic transducers, each pair of electromagnetic transducers comprising one shared electromagnetic transducer with at least one of the other pairs of electromagnetic transducers
receiving the tracked relative poses of the bone portions with respect to one another and compute real-time kinematics of the bone portions,
comparing the determined real-time kinematics with the recorded kinematic pattern of authorized displacements for each pair of electromagnetic transducers,
determining a difference between the real-time kinematics and the recorded kinematic pattern for each pair of electromagnetic transducers,
determining that the measurement of the electromagnetic field given by one pair of electromagnetic transducers is impaired when the difference exceeds a predetermined threshold,
determining which electromagnetic transducer gives impaired measurements based on the determination of which pair of electromagnetic transducers gives impaired measurements.

The method further comprises a step of indicating which electromagnetic transducer(s) gives the impaired measurements. The step of indicating which electromagnetic transducer(s) gives the impaired measurements comprises displaying a corresponding information or playing a vocal message.

The method further comprises a step of obtaining a corrected pose of the tracked bone portions by ignoring the impaired measurement while computing the real-time kinematic of the bone portions.

Other features and advantages of the invention will appear in the following description. Embodiments of the invention will be described with reference to the drawings, in which
- Figure 1 illustrates, schematically from a top view, a system for detecting the presence of electromagnetic disturbances according to the invention as it could be used during an osteotomy;
- Figure 2 illustrates the system of the invention associated with a surgical robotic system configured to treat a region of interest of an anatomical structure;
- Figure 3 is a diagram illustrating how different parts of the system of the invention interact with each other;
- Figure 4 illustrates, schematically, the system of the invention, in an embodiment wherein such system comprises at least three electromagnetic transducers and wherein the system is used during pose of pedicle screws
- Figure 5 and 6 schematically and partially illustrate two distinct recorded kinematic patterns of displacements of two bone portions linked to one another by a joint, the recorded kinematic pattern of figure 5 being used during a first phase of a surgery and the recorded kinematic pattern of figure 6 being used during a second phase of the same surgery.

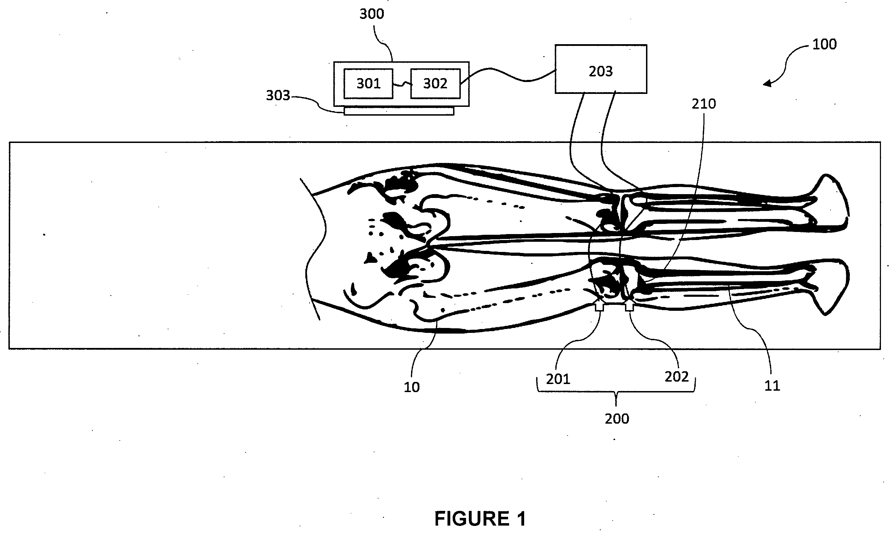

Figure 1 illustrates, schematically from a top view, a system 100 for determining that an electromagnetic disturbance is impairing measurements made by an electromagnetic tracking unit, according to the invention. Especially, figure 1 illustrates the system 100 of the invention used during an osteotomy. An osteotomy consists in cutting part of a tibia 11 or of a femur 10 of a patient so as to realign the femur 10 with respect to the tibia 11, thus relieving pressure on one compartment of the knee joint joining said femur 10 to said tibia 11. Once that the cut 210 is realized, the concerned bone portions are distracted or closed to achieve the realignment of the femur with respect to the tibia. The realization of the cut 210 thus creates an artificial joint linking two distinct portions of the same bone. In the present specification, the words "bone portions" refer to either an entire bone or to distinct portions of the same bone, and the word "joint" refers to either an anatomical joint such as a knee, a hip, an ankle or a shoulder for instance, or to an artificial joint such as the one created during an osteotomy.

The cut to be performed is generally planned based on images of the knee to be treated. In order to ensure that the planning is followed, the femur 10 and/or the tibia 11 must be tracked, so that their poses are known at any time. In the present specification, the word "pose" is used to refer to both a position and an orientation of the concerned object. According to the invention, this tracking is realized by an electromagnetic tracking unit.

The system 100 of this invention thus comprises the electromagnetic tracking unit 200 and a warning device 300. According to the embodiment illustrated on figure 1, the electromagnetic tracking unit 200 is configured to track, at least, the relative pose of the femur 10 with respect to the tibia 11. As well known in the art, such an electromagnetic tracking unit 200 tracks poses based on measurements of an electromagnetic field. Especially, the electromagnetic tracking unit 200 comprises at least two electromagnetic transducers, 201, 202 among which at least one electromagnetic transducer is configured to emit at least one electromagnetic field and at least one electromagnetic transducer is configured to receive and measure the emitted electromagnetic field.

According to the illustrated embodiment, the system comprises a first electromagnetic transducer 201 attached to the femur 10 and a second electromagnetic transducer 202 attached to the tibia 11. Figure 1 particularly illustrates a tibial osteotomy, i.e. an osteotomy wherein the cut 210 is realized on the tibia 11. Advantageously, a third electromagnetic transducer - not shown - can be attached on the tibia 11, the second electromagnetic transducer and the third electromagnetic transducer thus permitting to track the pose of the two bone portions linked to one another by the artificial joint created by the cut, therefore permitting to track the distraction or the closure once that the bone is cut. Alternatively, the first electromagnetic transducer and the second electromagnetic transducer could be both attached to the tibia or to the femur, on both sides of the planned cut, the electromagnetic tracking system thus only permitting to track the pose of the two bone portions linked to one another by the artificial joint created by the cut.

The electromagnetic transducers 201, 202 are schematically illustrated as arrows, a pic of each arrow indicating a potential location of the electromagnetic transducer with respect to the concerned bone portions. Optionally, the first and second electromagnetic transducers 201, 202 could be both electromagnetic receivers, the electromagnetic tracking unit thus comprising at least one electromagnetic transmitter configured to emit the electromagnetic field.

The electromagnetic tracking unit 200 further comprises a control unit 203 adapted to • determine the pose of the bone portions to which the electromagnetic transducers 201, 202 are attached based on the measurements of the electromagnetic field and on a known geometry between the concerned transducers and the bone portions to which they are attached. The control unit can, for instance, comprise one or more microprocessor, one or more' random access memory (RAM) and/or one or more read-only memory (ROM), one or more calculators, one or more computers and/or one or more computer programs.

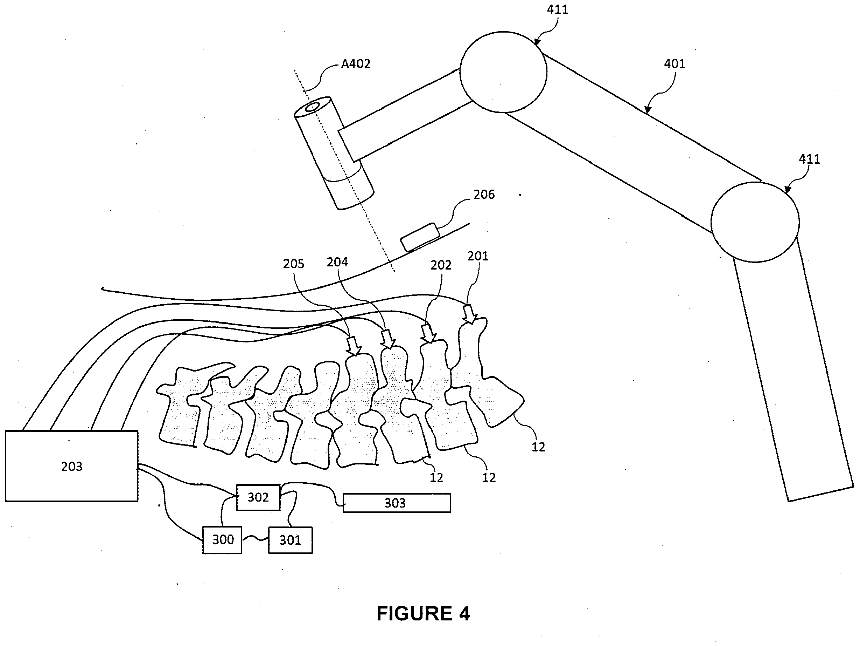

The warning device 300 comprises at least one storage unit 301 in which at least one kinematic pattern of authorized relative displacements of the tracked bone portions with respect to one another is recorded. The storage unit 301 can comprise one or more random access memory (RAM) and/or one or more read-only memory (ROM). The kinematic pattern can be determined pre-operatively and/or per-operatively. In the present specification, the word "pre-operatively" refers to any time before the patient enters the operating room, while the word "per-operatively" refers to any time after the patient enters the operating room. Alternatively, as shown on figure 4, the warning device 300 and the storage unit 301 could be two distinct components in communication with one another without departing from the scope of the invention.

In the present document, "the kinematic pattern of authorized relative displacements" refers to a set of successive authorized poses of the tracked bone portions with respect to one another. Therefore, the kinematic pattern of authorized relative displacements can also be seen as a set of authorized geometric configurations of two bone portions mobile around a common joint. It is understood that the poses of the bone portions are directly related to the poses of the corresponding electromagnetic transducers attached to each of these bone portions.

According to a first embodiment of the invention, the kinematic pattern of authorized relative displacements is recorded based on an *a priori* model applied to the particular geometry of the tracked bone portions of the concerned patient. According to this first embodiment, a plurality of 2D images of the tracked bone portions are taken and segmented. Segmenting an image consists in identifying, manually or automatically, the different structures, and particularly bony structures, present on the image. The segmented images are then used to build a 3D model of the bone portions and of the joint(s) formed between said bone portions. The 3D model can then be used to determine a range of authorized relative displacements of the tracked bone portions with respect to one another. This determination can for instance be realized based on a virtual simulation of relative displacements of the concerned bone portions. The kinematic pattern of authorized relative displacements can thus be recorded in the storage unit 301, pre-operatively. Obviously, the virtual simulation could also be realized per-operatively within the scope of the invention. According to another alternative, the 2D images can be taken pre-operatively while the virtual simulation can be realized per-operatively. According to this first embodiment, the kinematic pattern of authorized relative displacements is thus recorded based on a 3D model of the tracked bone portions. Thie kinematic pattern of authorized relative displacements is therefore a patient-specific pattern.

According to a second embodiment of the invention, the kinematic pattern of -authorized relative displacements can be recorded per-operatively, through a learning phase during which the surgeon, or any other user of the system, moves the tracked bone portions equipped with the electromagnetic transducers and simultaneously records the corresponding motions, thus resulting in recording the kinematic pattern of authorized relative displacements of the tracked bone portions with respect to each other. Obviously, the surgeon, or any other user of the system, must ensure that no disturbing material is present in the vicinity of the system while recording the kinematic pattern. According to this second embodiment, the surgeon must follow a predetermined list of movements to realize during the recording. Obviously, this second embodiment could be implemented pre-operatively within the scope of the invention. According to this second embodiment, the kinematic pattern of authorized relative displacements is thus recorded as authorized relative displacements of the electromagnetic transducers. As for the first embodiment, the kinematic pattern of authorized relative displacements is therefore a patient-specific pattern.

Obviously, the first and second embodiment could be combined. For instance, in the case of an osteotomy, a first kinematic pattern of authorized relative displacements between the femur and the tibia could be recorded according to the second embodiment, while a second kinematic pattern of authorized relative displacements of the portions of the bones which are planned to be distracted or closed once that the concerned bone is cut, can be recorded according to the first embodiment. The obtained first and second kinematic patterns can then be combined to obtain an overall kinematic pattern of authorized relative displacements for the whole surgery.

Additionally, several distinct kinematic patterns can be recorded and used during successive phases of the surgery. Such a configuration can for instance be useful during an osteotomy. During such an intervention, a first recorded kinematic pattern of authorized displacements can be used during a first phase during which the surgeon performs the cut, and a second recorded kinematic pattern of authorized displacements can be used during a second phase during which the surgeon distracts or closes the realized cut. Obviously, the authorized displacements of the concerned bone portions can be different depending on the phase of the surgery.

Regardless the embodiment implemented, the recorded kinematic pattern of authorized displacements can be regarded as several parametric shapes formed as successive authorized poses of each of the electromagnetic transducers. Thus, the recorded kinematic pattern is formed as several parametric shapes, each of these parametric shapes being associated with one of the electromagnetic transducers. For instance, the parametric shape can be formed as a portion of a sphere, or a portion of a cylinder, or a curved line...

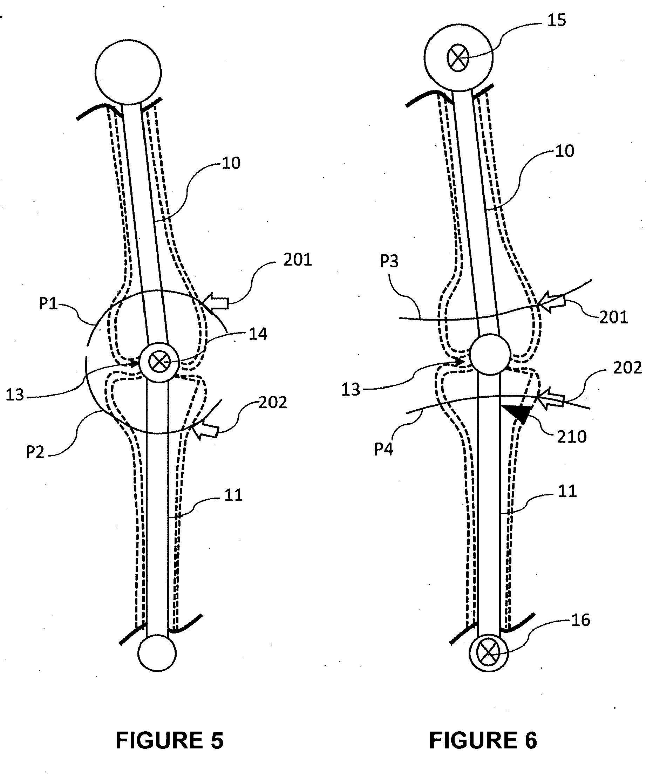

As for instance shown on figure 5, during a tibial osteotomy, the first electromagnetic transducer 201 is attached to the femur 10 and the second electromagnetic transducer 202 is attached to the tibia 11, the femur 10 and the tibia 11 being linked to one another by the knee joint 13. As previously described, an osteotomy aims at modifying the alignment of the tibia with respect to the femur to relieve pressure from the knee joint and can be divided into two major phases: a first phase consisting in performing the cut and a second phase consisting in distracting or closing the realized cut.

According to this particular embodiment, two distinct recorded kinematic patterns of authorized displacements can be recorded, each of which comprising a set of parametric shapes defined for each electromagnetic transducer.

According to the illustrated embodiment, a first recorded kinematic pattern of authorized displacements used during the first phase of the surgery comprises, at least, a first parametric surface P1 associated with the first electromagnetic transducer 201 and a second parametric surface P2 associated with the second electromagnetic transducer 202, both these parametric surfaces being defined around a centre formed by a knee centre 14. Figure 5 is a 2D representation, but it is understood that the first and the second parametric surfaces P1, P2, are formed as portions of spheres. Once that the cut 210 is realized, the surgeon or any other user of the system can indicate that the first phase is completed and that the second phase is about to start so as for the data processor to switch from the first kinematic pattern of authorized displacements schematically and partially illustrated on figure 5 to a second kinematic pattern of authorized displacements schematically and partially illustrated on figure 6.

Figure 6 thus illustrates part of the second recorded kinematic pattern of authorized displacements used during the second phase of the surgery. As partially illustrated, the second kinematic pattern of authorized displacements comprises, at least, a third parametric surface P3 associated with the first electromagnetic transducer 201 and a fourth parametric surface P4 associated with the second electromagnetic transducer 202, the third parametric surface being defined around a centre formed by a hip centre 15 and the fourth parametric surface being defined around a centre formed by an ankle centre 16. Again, figure 6 is a 2D representation of the parametric surfaces which are actually 3D parametric shapes.

Referring back to figure 1, the warning device 300 further comprises at least one data processor 302. The data processor 302 is in communication with, at least, the storage unit 301 of the warning device 300 and the electromagnetic tracking unit 200. According to the invention, the data processor 302 can communicate with the storage unit and with the electromagnetic tracking unit through a wired connection - as schematically illustrated - or wirelessly. Optionally, the warning device 300 can comprise a display 303. Again, as for instance illustrated on figure 4, the data processor 302, the warning device 300 and/or the display 303 could be distinct components in communication with each other within the scope of the invention.

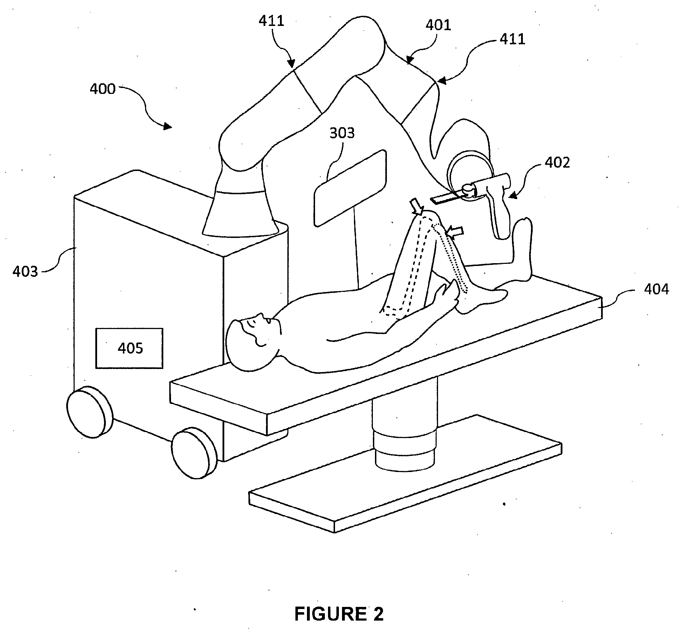

Optionally, as for instance shown on figure 2, the system 100 of the invention can be used with a surgical robotic system 400. As shown, such a surgical robotic system 400 can for instance comprise a robotic arm 401 holding a surgical tool 402 adapted to perform a planned treatment. According to the illustrated embodiment, the robotic arm 401 is mounted on a wheeled cart 403, but it could be directly fixed to the ground, or to a surgical table 404 within the scope of the invention. The planned treatment comprises a surgical plan which defines which anatomical structure(s) must be treated and how they should be treated. Such surgical robotic systems can be associated with the electromagnetic tracking systems in order to locate, in real-time, the surgical tool with respect to the anatomical structure to be treated. The surgical robotic system 400 further comprises at least one controller 405, schematically illustrated as embedded within the wheeled cart 403, configured to instruct displacements to motorized joints 411 of the robotic arm 401. The surgical robotic system 400 can be controlled by a surgeon, tele-operatively or not, or it could be totally autonomous without departing from the scope of the invention. The controller 405 and the data processor 302 can be formed as a single unit or they can be separated components in communication with each other within the scope of the invention. Data processor 302 is therefore not illustrated on figure 2.

According to the illustrated example which represents an osteotomy procedure, the surgical tool 402 is a surgical saw. Obviously, the surgical tool could be of any kind depending on the surgery to perform. For instance, it could be a burr, or a screwdriver.

For an osteotomy, the planned treatment comprises a surgical plan which defines the kind of osteotomy to be performed. By "kind of osteotomy", we here mean that it can be an open-wedge osteotomy or a closed-wedge osteotomy depending on the compartment of the knee on which the pressure must be decreased. Also, the words "kind of osteotomy" refer to either a femoral osteotomy or a tibial osteotomy.

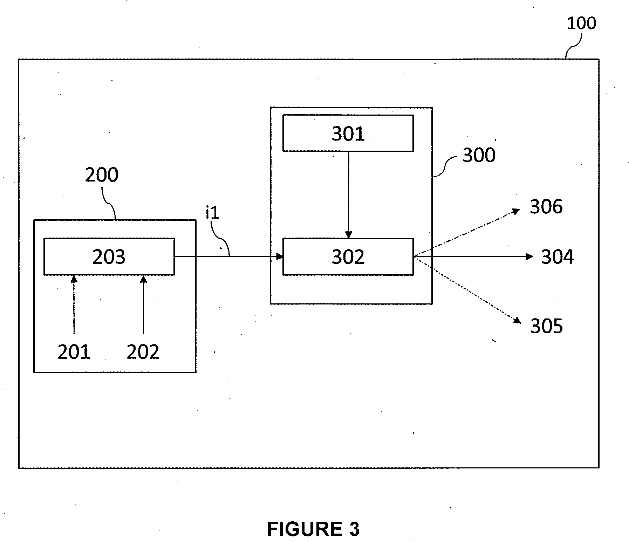

We are now going to describe how the described different parts of the system of the invention interact with each other with reference to Figure 3 which is a diagram. As shown, the data processor 302 is configured to receive an information i1 from the electromagnetic tracking unit 200, related to the tracked relative poses of the bone portions with respect to one another. The data processor 302 can then compute a real-time kinematic of the bone portions, based on the received relative poses and on the known geometry between the electromagnetic transducers and the bone portions to which they are attached. In the present specification, the words "real-time kinematic of the bone portions" refer to a computation of the geometry of the tracked bone portions with respect to one another at a given time.

The data processor 302 then compares the real-time kinematic of the bone portions with the kinematic pattern recorded in the storage unit 301 and determine a difference between the real-time kinematic and the recorded kinematic pattern. The determined difference is then compared to a predetermined threshold, this threshold being recorded in the storage unit 301 too. The data processor 302 is then configured to determine that at least one of the measurements of the electromagnetic field is impaired when the difference exceeds the predetermined threshold.

When it is determined that at least one of the measurements of the electromagnetic field is impaired, the data processor 302 is configured to trigger a warning signal 304 generated by the warning device 300. This warning signal 304 can be of any kind within the scope of the invention. For instance, it can be a warning sound, a warning light, a beacon or even vibrations or any other haptic signal. Thus, the warning device 300 is also equipped with means for generating said light, sound and/or vibrations.

When the system 100 of the invention is associated to a surgical robotic system as described above, the data processor 303 can be further adapted to trigger the warning signal and/or to trigger an emergency stop 305, preventing that the robotic arm, and especially the surgical tool held by this robotic arm, harms the patient, which could occur if the electromagnetic tracking system is not reliable anymore.

The data processor can, for instance, comprise one or more microprocessor, one or more calculators, one or more computers and/or one or more computer programs. The computer program(s) comprise code instructions to, at least, compute the real-time kinematic of the bone portions, compare the real-time kinematic of the bone portions with the recorded kinematic pattern, determine the difference between the real-time kinematic and the recorded kinematic pattern and determine that at least one of the measurements of the electromagnetic field is impaired when the difference exceeds a predetermined threshold.

The predetermined threshold can be modified depending on the treatment to be performed. Advantageously, this predetermined threshold is set to be similar to the expected accuracy for the treatment. For instance, if the accuracy expected for the treatment is of a tenth of millimeter; then a difference greater than this between the real-time kinematic of the tracked bone portions and the recorded kinematic pattern will be considered as reflecting an impaired measurement by the data processor.

When the electromagnetic tracking system comprises at least three transducers attached to three bone portions linked to one another by at least two joints - for instance distributed on adjacent vertebrae as illustrated on figure 4 or distributed as described above for an osteotomy procedure - the data processor 302 can be configured to determine which electromagnetic transducer(s) give the impaired measurements based on the determined difference between the real-time kinematic and the recorded kinematic pattern. According to this aspect of the present invention, the data processor 302 is configured to compare and determine the difference between the recorded kinematic pattern of authorized displacements with the determined real-time kinematic of the bone portions, two by two, thus artificially creating two pairs of electromagnetic transducers with only one shared electromagnetic transducer. Thus, if the difference exceeds the predetermined threshold only for one of the pairs of electromagnetic transducers, the data processor is configured to determine that the non-shared electromagnetic transducer of the concerned pair gives impaired measurement.

Figure 4 for instance illustrates this aspect of the invention. Especially, figure 4 illustrates, schematically, the pose of pedicle screws. Such a surgery consists in positioning screws in the pedicles of selected vertebrae 12 - only three of them being referenced on figure 4 - for instance to help healing a fracture of the rachis, or to help correct a twisted rachis, as it can be seen for patient with scoliosis. Figure 4 particularly illustrates the system of the invention used with a robotic system 400 which holds a surgical guide 406 configured to guide, successively, at least, a drill and a screwdriver. The drill and the screwdriver are schematically illustrated by an axis A402 representing the axis along which these surgical tools are guided. According to the illustrated embodiment, the electromagnetic tracking unit 200 comprises one electromagnetic transmitter 206 arranged in a fixed position on the skin of the patient, this electromagnetic transmitter 206 being configured to emit at least one electromagnetic field, and four electromagnetic receivers 201, 202, 204, 205, configured to receive and measure the emitted electromagnetic field, are attached, respectively, to four adjacent vertebrae.

According to this illustrated embodiment, the data processor can advantageously be configured to compare, and determine the difference between the recorded kinematic pattern of the adjacent vertebrae 12, with the determined real-time kinematic of these adjacent vertebrae, two by two, thus artificially creating four pairs of electromagnetic receivers, each pair of electromagnetic receivers sharing one electromagnetic transducer with at least one of the other pairs. If the determined difference between the real-time kinematic and the recorded kinematic pattern exceeds the predetermined threshold only for two pairs of electromagnetic transducers which share one electromagnetic receiver, the data processor is configured to determine that only this shared electromagnetic receiver gives impaired measurements.

Once that the data processor 302 has determined which electromagnetic transducer(s) give the impaired measurements, this data processor 302 can then be configured to display a corresponding information on the display 303. Alternatively or cumulatively, the data processor 302 can be configured to play a vocal message indicating which electromagnetic transducer gives impaired measurements. For instance, a number, or a colour can be assigned to each electromagnetic transducer so that the user of the system 100 can quickly identify which electromagnetic transducer gives impaired measurements.

Referring back to figure 3, the data processor 302 can also optionally be configured to compute corrected poses 306 of the tracked bone portions. For instance, the data processor 302 can be configured to automatically ignore the impaired measurements in order to obtain a corrected pose of the electromagnetic transducers. In this case, the data processors can be configured to replace the received pose of the electromagnetic transducer identified as giving impaired measurements by its theoretical pose, recorded with the poses of the other electromagnetic transducers in the recorded kinematic pattern of authorized relative displacements.

The given examples of surgery for which the system of the invention could be used is only intended to illustrate the invention, not limiting it. It is understood, based on the present specification, that the system of the invention could be used in any other kind of orthopedic surgery within the scope of the invention. For instance, it could be used during a total or partial knee arthroplasty, a hip replacement, an ankle replacement of even a knee ligamentoplasty.

## Claims

1. System (100) for determining that an electromagnetic disturbance is impairing measurements made by an electromagnetic tracking unit (200) during a surgery, the system (100) comprising
the electromagnetic tracking unit (200) configured to track relative poses of at least two bone portions (10, 11, 12) linked to one another by at least one joint (13), based on measurements of an electromagnetic field emitted and received, respectively, by at least two electromagnetic transducers (201, 202, 204, 205) of the electromagnetic tracking unit (200),
at least one storage unit (301) in which at least one kinematic pattern of authorized relative displacements of the tracked bone portions (10, 11, 12) with respect to one another is recorded, and
one data processor (302) in communication with the storage unit (301) and with the electromagnetic tracking unit (200),
wherein the data processor (302) is configured to
receive the tracked relative poses of the bone portions (10, 11, 12) with respect to one another and compute a real-time kinematic of the bone portions (10, 11, 12),
compare the real-time kinematic of the bone portions (10, 11, 12) with the recorded kinematic'pattern,
determine a difference between the real-time kinematic and the recorded kinematic pattern,
determine that at least one of the measurements of the electromagnetic field is impaired when the difference exceeds a predetermined threshold.

2. System (100) according to claim 1, comprising at least one warning device (300) in communication with the data processor (302), wherein the data processor (302) is further configured to trigger a warning signal generated by the warning device (300), when it is determined that at least one of the measurements of the electromagnetic field is impaired.

3. System (100) according to any of the preceding claims, wherein the electromagnetic tracking unit (200) is configured to track the relative poses of at least three bone portions (10, 11, 12) linked to one another by at least two joints, and wherein the data processor (302) is further configured to determine which electromagnetic transducer (201, 202, 204, 205) gives impaired measurement(s).

4. System (100) according to claim 3, wherein the data processor (302) is configured to determine which electromagnetic transducer (201, 202, 204, 205) gives impaired measurement(s) by
artificially creating pairs of electromagnetic transducers (201, 202, 204, 205), each pair of electromagnetic transducers (201, 202, 204, 205) comprising one shared electromagnetic transducer (201, 202, 204, 205) with at least one of the other pairs of electromagnetic transducers (201, 202, 204, 205)
receiving the tracked relative poses of the bone portions (10, 11, 12) with respect to one another and compute real-time kinematics of the bone portions (10, 11, 12),
comparing the determined real-time kinematics with the recorded kinematic pattern of authorized displacements for each pair of electromagnetic transducers (201, 202, 204, 205),
determining a difference between the real-time kinematics and the recorded kinematic pattern for each pair of electromagnetic transducers (201, 202, 204, 205),
determining that the measurement of the electromagnetic field given by one pair of electromagnetic transducers (201, 202, 204, 205) is impaired when the difference exceeds a predetermined threshold,
determining which electromagnetic transducer (201, 202, 204, 205) gives impaired measurements based on the determination of which pair of electromagnetic transducers (201, 202, 204, 205) gives impaired measurements.

5. System (100) according to claim 3 or 4, comprising at least one display (303) and wherein the data processor (302) is configured to display which electromagnetic transducer (201, 202, 204, 205) gives impaired measurements.

6. System (100) according to any of claims 3 to 5, wherein the data processor (302) is configured to compute a corrected relative pose of the bone portions (10, 11, 12) by ignoring the impaired measurement(s).

7. System (100) according to any of the preceding claims, wherein the kinematic pattern of authorized relative displacements is recorded based on a 3D model of the tracked bone portions (10, 11, 12).

8. System (100) according to any of the preceding claims, wherein the kinematic pattern of authorized relative displacements is recorded as authorized relative displacements of the electromagnetic transducers (201, 202, 204, 205).

9. System (100) according to any of the preceding claims, wherein the electromagnetic tracking unit (200) comprises one electromagnetic transmitter (206) adapted to emit the electromagnetic field and at least two electromagnetic receivers (201, 202, 204, 205) adapted to receive and measure the emitted electromagnetic field, each of the electromagnetic receivers being configured to be attached, respectively, to one of the tracked bone portions (10, 11, 12).

10. Surgical robotic system (400) comprising, at least
a robotic arm (401) comprising at least one motorized joint (13), the robotic arm (401) being configured to hold a surgical tool (402),
the surgical tool (402) configured to treat a region of interest of an anatomical structure,
the system (100) for determining that an electromagnetic disturbance is impairing measurements made by the electromagnetic tracking unit according to any of the preceding claims.

11. Surgical robotic system (400) according to the preceding claim, wherein the data processor (302) of the system (100) for determining that an electromagnetic disturbance is impairing measurements made by the electromagnetic tracking unit (200) is further configured to trigger an emergency stop of the robotic arm (401) when it is determined that at least one of the measurements of the electromagnetic field is impaired.
